# EUROPEAN PATENT SPECIFICATION

(11) **EP 3 216 476 B1**
(45) Date of publication and mention of the grant of the patent: **13.03.2019**
(21) Application number: 17173856.0
(22) Date of filing: 31.05.2017
(51) Int. Cl.: A61M 15/06, A24F 47/00, B05B 11/00, H05B 3/00, A61M 11/00, A61M 11/04

(54) **SPRAYING ATOMIZING DEVICE**
ZERSTÄUBER-SPRÜHVORRICHTUNG
DISPOSITIF D'ATOMISATION DE PULVÉRISATION

(30) Priority: 03.06.2016 CN 201610385852
(43) Date of publication of application: 13.09.2017
(73) Proprietor: Shenzhen First Union Technology Co., Ltd., Shenzhen, Guangdong 518104 (CN)
(72) Inventor: LI, Yonghai, Shenzhen, Guangdong 518104 (CN); XU, Zhongli, Shenzhen, Guangdong 518104 (CN); HU, Shuyun, Shenzhen, Guangdong 518104 (CN)
(74) Representative: Proi World Intellectual Property GmbH

(56) References cited:
- EP-A1- 0 845 220
- EP-A2- 2 172 239
- WO-A1-2013/089358
- WO-A1-2016/033715
- WO-A2-2007/000330
- CN-A- 102 166 044
- CN-A- 104 126 876
- CN-A- 105 192 891
- CN-U- 204 048 045
- CN-U- 204 157 647
- US-A- 5 261 949
- US-A- 5 666 977
- US-A1- 2005 016 550
- US-A1- 2005 042 170

## Description

### TECHNICAL FIELD

The present invention relates to electronic cigarettes, and particularly to a spraying atomizing device.

### BACKGROUND ART

An electronic cigarette includes a glass fiber core, and a heating wire in contact with the glass fiber core. The heating wire is configured for heating tobacco liquid to atomize. However, when the tobacco liquid is boiled, liquid particles of large size may be generated and inhaled by the user, rendering user unsatisfactory.

What is needed, therefore, is a spraying atomizing device, which can overcome the above shortcomings.

Different prior art spraying atomizing devices are known, for example, from the documents EP 0 845 220 A1, US 2005/016550 A1, US 5 666 977 A, EP 2 172 239 A2, WO 2007/000330 A2, CN 105 192 891 A, CN 204 157 647 U, CN 104 126 876 A, WO 2013/089358 A1, CN 204 048 045 U and WO 2016/033715 A1.

### SUMMARY

The present invention provides a spraying atomizing device as defined in claim 1.

An exemplary spraying atomizing device includes a housing, a liquid tank, a spraying device, a heating element, and a power supply. The housing defines an atomizing chamber. The liquid tank is configured for storing tobacco liquid. The spraying device is configured for spraying the tobacco liquid into the atomizing chamber in a form of liquid particles. The heating element is arranged in the atomizing chamber, and has a heating surface. The heating surface is configured for supporting and heating the liquid particles to form aerosol. The power supply is arranged in the housing, and is configured for feeding the heating element power. The atomizing device further comprises a receiving chamber in the housing below the heating element, the receiving chamber being in communication with the liquid tank, so that the liquid particles, which are not atomized, can flow into the receiving chamber, and then into the liquid tank.

### BRIEF DESCRIPTION OF THE DRAWINGS

Many aspects of the present disclosure can be better understood with reference to the following drawings. The components in the drawings are not necessarily drawn to scale, the emphasis instead being placed upon clearly illustrating the principles of the present disclosure. Moreover, in the drawings, like reference numerals designate corresponding parts throughout the several views.
FIG. 1 is a cross-sectional view of a spraying atomizing device according to an embodiment.
FIG. 2 is a perspective view of a heating element of the atomizing device of FIG. 1.
FIG. 3 is a cross-sectional view of a spraying device of the atomizing device of FIG. 1.
FIG. 4 is an enlarged view of area A of FIG. 3.

### DETAILED DESCRIPTION

It will be appreciated that for simplicity and clarity of illustration, where appropriate, reference numerals have been repeated among the different figures to indicate corresponding or analogous elements. In addition, numerous specific details are set forth in order to provide a thorough understanding of the embodiments described herein. However, it will be understood by those of ordinary skill in the art that the embodiments described herein can be practiced without these specific details. In other instances, methods, procedures and components have not been described in detail so as not to obscure the related relevant feature being described. Also, the description is not to be considered as limiting the scope of the embodiments described herein. The drawings are not necessarily to scale and the proportions of certain parts have been exaggerated to better illustrate details and features of the present disclosure.

The disclosure is illustrated by way of example and not by way of limitation in the figures of the accompanying drawings in which like references indicate similar elements. It should be noted that references to "an" or "one" embodiment in this disclosure are not necessarily to the same embodiment, and such references mean at least one.

Several definitions that apply throughout this disclosure will now be presented.

The term "outside" refers to a region that is beyond the outermost confines of a physical object. The term "inside" indicates that at least a portion of a region is partially contained within a boundary formed by the object. The term "substantially" is defined to be essentially conforming to the particular dimension, shape or other word that substantially modifies, such that the component need not be exact. For example, substantially cylindrical means that the object resembles a cylinder, but can have one or more deviations from a true cylinder. The term "comprising," when utilized, means "including, but not necessarily limited to"; it specifically indicates open-ended inclusion or membership in the so-described combination, group, series and the like.

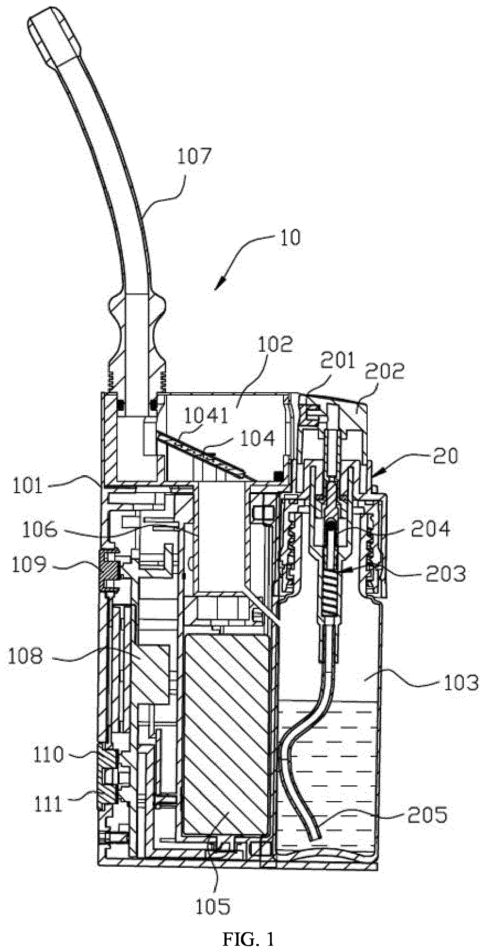

Referring to FIG. 1, a spraying atomizing device 10 is shown. The atomizing device 10 is configured (i.e., structured and arranged) for atomizing tobacco liquid to form aerosol. The atomizing device 10 includes a housing 101, a liquid tank 103, and a spraying device 20, a heating element 104, and a power supply 105. The spraying device 20, the heating element 104, and the power supply 105 are arranged in the housing 101.

The housing 101 defines an atomizing chamber 102 at a top part of the housing 101. The heating element 104 is arranged in the atomizing chamber 102. The spraying device 20 is configured for spraying tobacco liquid in a form of liquid particles into the atomizing chamber 102. The heating element 104 is configured for heating the liquid particles to form aerosol in a form of molecules. The spraying device 20 and the liquid tank 103 are interconnected to form an integral structure. In the present embodiment, the spraying device 20 and the liquid tank 103 may be arranged in the housing 101.

A mouthpiece 107 is provided on the housing 101. The mouthpiece 107 is in communication with the atomizing chamber 102. Quite usefully, the mouthpiece 107 and the spraying device 20 are arranged at two opposite sides of the heating element 104. The spraying device 20 sprays liquid particles into the atomizing chamber 102, the liquid particles are further atomized to aerosol in the atomizing chamber 102, and then the aerosol is expelled via the mouthpiece 107.

The liquid tank 103 is configured for storing tobacco liquid, and includes an open end. The spraying device 20 absorbs tobacco liquid from the liquid tank 103. The liquid tank 103 is connected to the spraying device 20 via the open end, for example, threadedly. When the liquid tank 103 is detached, tobacco liquid can be injected via the open end.

The power supply 105 is received in the housing 101, and is configured for supplying the heating element 104 power. The power supply 105 may be a lithium battery. The power supply 105 is electrically connected with the heating element 104 via a circuit board 108. A switch 109 is provided on the housing 101 adjacent to the circuit board 108. When the switch 109 is pressed, the heating element 104 works. Quite usefully, buttons 110, 111 are provided on the housing 101, and are configured for adjusting an output wattage of the power supply 105, e.g., increasing or decreasing the output wattage.

In the present embodiment, the heating element 104 includes a heating surface 1041. The heating surface 1041 is configured for supporting the liquid particles, and heating the liquid particles to atomize. The heating surface 1041 faces the spraying device 20, and is inclined relative to the horizontal direction. Referring to FIG. 1, in the present embodiment, an included angle between the heating surface 1041 and the horizontal direction is about 30 degrees. Accordingly, the heating element 104 can provide a large atomizing area, thus enhancing atomizing efficiency.

Quite usefully, a receiving chamber 106 is defined in the housing below the heating element 104. The receiving chamber 106 is in communication with the liquid tank 103 via a tube. Because the heating surface 1041 is inclined, liquid particles, which are not atomized, may flow into the receiving chamber 106, and then into the liquid tank 103, thus forming a circulatory system, and enhancing an atomizing efficiency. Further, liquid leakage is prevented from the atomizing chamber 102.

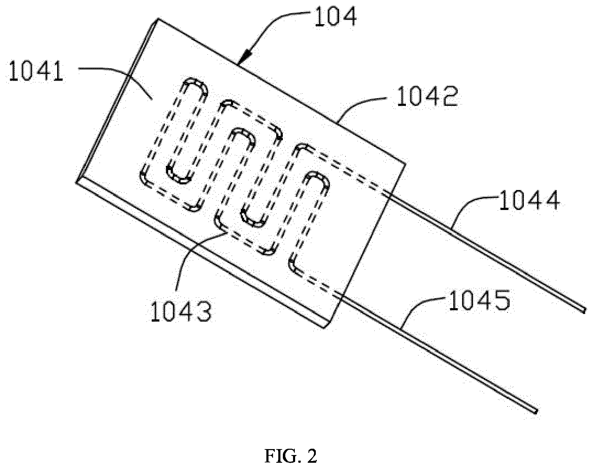

Referring to FIG. 2, the heating element 104 may be made of porous material, e.g., porous ceramic or porous metal. Quite usefully, the heating element 104 is made of porous ceramic. The heating element 104 includes a porous ceramic body 1042, a metallic heating part 1043, and two electrodes 1044, 1045. The heating part 1043 is embedded in the porous ceramic body 1042. Two opposite ends of the heating part 1043 are connected to the two electrodes 1044, 1045, respectively.

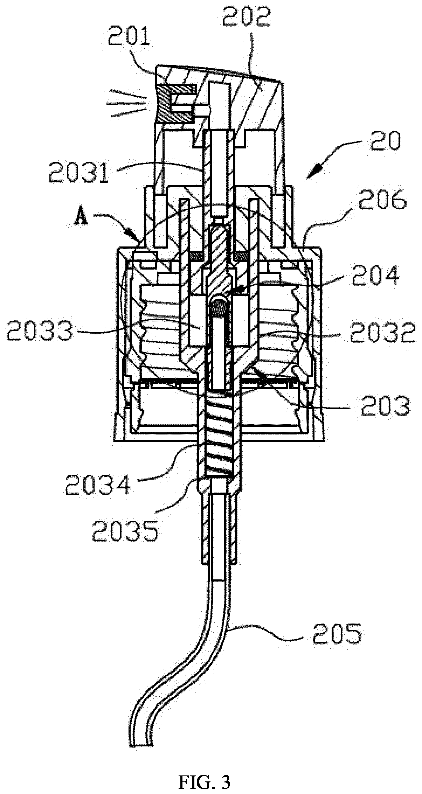

Referring to FIG. 3, the spraying device 20 includes a nozzle 201, a pressing head 202, a pump body 203, and a suction tube 205 connected to an end of the pump body 203. A bottom end of the suction tube 205 is immersed in the tobacco liquid. The pump body 203 is in communication with the tobacco liquid in the liquid tank 103 via the suction tube 205. An air pressure in the pump body 203 can be changed by pressing the pressing head 203, so that tobacco liquid can be sprayed via the nozzle 201 and atomized. The other end of the pump body 203 is connected to the nozzle 201. The pressing head 202 is movably arranged on the fixing holder 206. The pressing head 202 is capable of moving up and down along the fixing holder 206 to compress a space in the pump body 203, thus changing a pressure in the pump body 203. The liquid tank 103 is threadedly coupled to the fixing holder 206, and the nozzle 201 is arranged in the pressing head 202. The nozzle 201 is configured for spraying liquid particles horizontally into the atomizing chamber 102. An included angle is formed between a spraying direction of the nozzle 201 and the heating surface 1041 of the heating element 104. In this way, an area of secondary atomizing is increased. The included angle may be 30 degrees.

In the above embodiment, the atomizing device 10 includes a switch 109. In an alternative embodiment, a touch switch is provided on the housing 101 adjacent to the pressing head 202. When the pressing head 202 is pressed, the touch switch is touched simultaneously, thus activating the heating element 104.

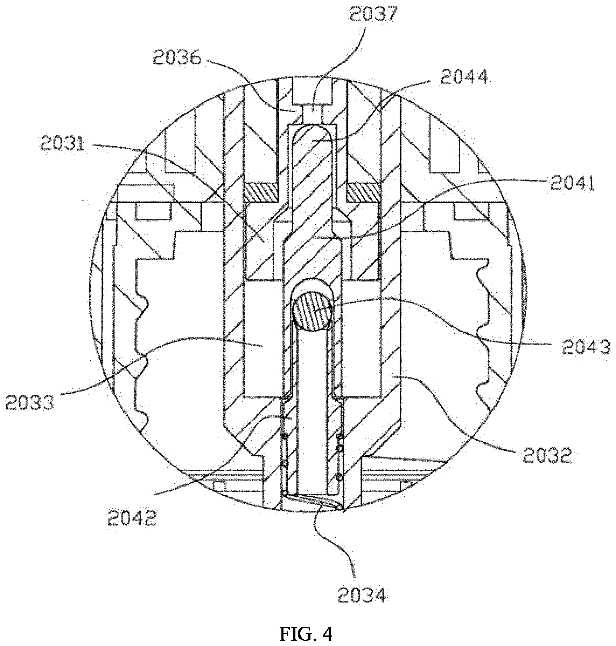

Referring to FIGS. 3-4, the pump body 203 includes a main body 2032 and a piston 2031 arranged in the main body 2032. The piston 2031 is hollow and is in communication with the mouthpiece 201. A top part of the piston 2031 is connected with the pressing head 202. The pressing head 202 can push the piston 202 to move downwards, and a sealing ring is provided between the piston and the main body 2032. A bottom part of the piston 2031 and the main body 2032 cooperatively define a compression chamber 2033. A valve body 204 is provided in the compression chamber 2033, and configured for controlling entrance of tobacco liquid into the compression chamber 2033. The main body 2032 is hollow. A bottom end of the main body is connected with the suction pipe 205. When the pressing head 202 is pressed, air or tobacco liquid in the compression chamber 2033 can be expelled via the nozzle 201 along an internal pipe of the piston 2031.

Further, the piston 2031 includes an annular stage 2036 protruding inwards. The annular stage 2036 defines a spraying hole 2037 of small diameter. A top part of the valve body 204 abuts against the annular stage 2036. A gap is defined between the annular stage 2036 and the spraying hole 2037. A spring 2034 is provided abutting against a bottom end of the valve body 204, and configured for driving the valve body 204 to restore to its initial position when the valve body 204 is moved downwards.

Quite usefully, the valve body 204 includes a top valve body 2041, a bottom valve body 2042, and a valve bead 2043. Part of the bottom valve body 2042 is nested in the top valve body 2041. The valve bead 2043 is sandwiched between the top valve body 2041 and the bottom valve body 2042. The bottom valve body 2042 is hollow and in communication with the suction tube 205. The top valve body 2041 and the bottom valve body 2042 cooperatively define a gap. The gap allows tobacco liquid to flow through. A top part 2044 of the top valve body 2041 abuts against the annular stage 2036. The top valve body 2041 and the bottom valve body 2042 are movable along an axial direction of an internal chamber of the main body 2032. A step portion 2035 is formed in the internal chamber of the main body 2032. Atop end of the spring 2034 abuts against the bottom valve body 2042, and a bottom end of the spring 2034 abuts against the step portion 2035.

The working principle of the pump body 204 will be described in detail below. When the pressing head 202 is pressed, the piston 2031, the top valve body 2041 and the bottom valve body 2042 are driven to move downwards, the spring 2034 is compressed, and air in the compression chamber 2033 is compressed. The gap between the top valve body 2041 and the bottom valve body 2042 is blocked by the valve bead 2043. The air in the compression chamber 2033 cannot be expelled through the gap between the top valve body 2041 and the bottom valve body 2042, and can only be expelled via the spraying hole 2037. When the pressing head 202 is released, a negative pressure occurs in the compression chamber 2033, the spring 2034 drives the top valve body 2041, the bottom valve body 2042, and the piston 2031 to move upwards. The tobacco liquid in the liquid tank 103 flows through the suction tube 205, the internal chamber of the main body 2032, an internal chamber of the bottom valve body 2042, the gap between the top valve body 2041 and the bottom valve body 2942, and is finally sucked into the compression chamber 2033.

When the pressing head 202 is pressed again, the air in the compression chamber 2033 is compressed again, an air pressure in the compression chamber 2033 is increased, and the tobacco liquid in the compression chamber 2033 is forced to expel via a gap between the top part 2044 of the top valve body 2041 and the spraying hole 2037 in a form of fog. The tobacco liquid is finally sprayed into the atomizing chamber 102 via the nozzle 201.

It is understood that the above-described embodiments are intended to illustrate rather than limit the disclosure. Variations may be made to the embodiments without departing from the scope of the present invention as defined by the following appended claims.

## Claims

1. A spraying atomizing device (10), comprising:
a housing (101), the housing (101) defining an atomizing chamber (102);
a liquid tank (103) configured for storing tobacco liquid;
a spraying device (20) configured for spraying the tobacco liquid into the atomizing chamber (102) in a form of liquid particles;
a heating element (104) arranged in the atomizing chamber (102), the heating element (104) having a heating surface (1041), the heating surface (1041) being configured for supporting and heating the liquid particles to form aerosol; and
a power supply (105) arranged in the housing (101), the power supply (105) being configured for feeding the heating element power;
**characterised in that** the atomizing device (10) further comprises a receiving chamber (106) in the housing (101) below the heating element (104), wherein the receiving chamber (106) is in communication with the liquid tank (103), so that the liquid particles, which are not atomized, can flow into the receiving chamber (106), and then into the liquid tank (103).

2. The atomizing device according to claim 1, wherein the heating surface (1041) faces the spraying device (20), and is inclined relative to a horizontal direction.

3. The atomizing device according to claim 1, wherein the spraying device (20) comprises a nozzle (201), a pressing head (202), a pump body (203), and a suction tube (205) connected to an end of the pump body (203), the pump body (203) is in communication with the tobacco liquid in the liquid tank (103) via the suction tube (205), an air pressure in the pump body (203) can be changed by pressing the pressing head (202), such that tobacco liquid can be sprayed and atomized via the nozzle (201).

4. The atomizing device according to claim 3, wherein the pump body (203) comprises a main body (2032), a piston (2031) in the main body (2032), and a valve body (204), the piston (2031) is hollow and in communication with the nozzle (201), the piston (2031) and the main body (2032) cooperatively define a compression chamber (2033), the valve body (204) is arranged in the compression chamber (2033), and configured for controlling entrance of tobacco liquid into the compression chamber (2033).

5. The atomizing device according to claim 4, wherein the pump body (203) further comprises an annular stage (2036) and a spring (2034), the annular stage (2036) forms a spraying hole (2037), a top end (2044) of the valve body (204) abuts against the annular stage (2036), and a gap is formed between the annular stage (2036) and the spraying hole (2037), and a bottom end of the valve body (204) abuts against the spring (2034).

6. The atomizing device according to claim 4, wherein the valve body (204) comprises a top valve body (2041), a bottom valve body (2042), and a valve bead (2043), part of the bottom valve body (2042) being nested in the top valve body (2041), the valve bead (2043) is sandwiched between the top valve body (2041) and the bottom valve body (2042), the bottom valve body (2042) is hollow and in communication with the suction tube (205), the top valve body (2041) and the bottom valve body (2042) cooperatively define a gap.

7. The atomizing device according to claim 1, wherein the heating element (104) is made of porous ceramic or porous metal.

8. The atomizing device according to claim 1, further comprising a mouthpiece (107), wherein the mouthpiece (107) and the spraying device (20) are arranged at two opposite sides of the heating element (104).

9. The atomizing device according to claim 1, further comprising a button (110 ; 111) configured for adjusting an output wattage of the power supply (105).

## Patentansprüche

1. Sprühzerstäubungsvorrichtung (10), umfassend:
ein Gehäuse (101), wobei das Gehäuse (101) eine Zerstäuberkammer (102) definiert;
einen Flüssigkeitstank (103), der zum Speichern von Tabakflüssigkeit konfiguriert ist;
eine Sprühvorrichtung (20), die zum Sprühen der Tabakflüssigkeit in die Zerstäubungskammer (102) in Form von Flüssigkeitsteilchen konfiguriert ist;
ein Heizelement (104), das in der Zerstäubungskammer (102) angeordnet ist, wobei das Heizelement (104) eine Heizfläche (1041) aufweist, wobei die Heizfläche (1041) zum Tragen und Erwärmen der Flüssigkeitsteilchen zur Bildung von Aerosol konfiguriert ist; und
eine Stromversorgung (105), die in dem Gehäuse (101) angeordnet ist, wobei die Stromversorgung (105) zum Einspeisen der Heizelementleistung konfiguriert ist;
**dadurch gekennzeichnet, dass**
die Sprühzerstäubungsvorrichtung (10) ferner eine Aufnahmekammer (106) in dem Gehäuse (101) unter dem Heizelement (104) umfasst, wobei die Aufnahmekammer (106) mit dem Flüssigkeitstank (103) in Verbindung steht, so dass die Flüssigkeitsteilchen, die nicht zerstäubt werden, in die Aufnahmekammer (106) fließen können, und dann in den Flüssigkeitstank (103).

2. Zerstäubungsvorrichtung nach Anspruch 1, wobei die Heizfläche (1041) der Sprühvorrichtung (20) zugewandt ist und relativ zu einer horizontalen Richtung geneigt ist.

3. Zerstäubungsvorrichtung nach Anspruch 1, wobei die Sprühvorrichtung (20) eine Düse (201), einen Druckkopf (202), einen Pumpenkörper (203) und ein Saugrohr (205) umfasst, das mit einem Ende des Pumpenkörpers (203) verbunden ist, wobei der Pumpenkörper (203) mit der Tabakflüssigkeit in dem Flüssigkeitstank (103) über das Saugrohr (205) in Verbindung steht, wobei ein Luftdruck im Pumpenkörper (203) durch Drücken des Presskopfes (202) verändert werden kann, so dass Tabakflüssigkeit über die Düse (201) versprüht und zerstäubt werden kann.

4. Zerstäubungsvorrichtung nach Anspruch 3, wobei der Pumpenkörper (203) einen Hauptkörper (2032), einen Kolben (2031) im Hauptkörper (2032) und einen Ventilkörper (204) umfasst, der Kolben (2031) hohl ist und mit der Düse (201) in Verbindung steht, wobei der Kolben (2031) und der Hauptkörper (2032) zusammenwirkend eine Kompressionskammer (2033) definieren, wobei der Ventilkörper (204) in der Kompressionskammer (2033) angeordnet und dazu konfiguriert ist, den Eintritt von Tabakflüssigkeit in die Kompressionskammer (2033) zu steuern.

5. Zerstäubungsvorrichtung nach Anspruch 5, wobei der Pumpenkörper (203) ferner eine ringförmige Stufe (2036) und eine Feder (2034) umfasst, die ringförmige Stufe (2036) ein Sprühloch (2037) bildet, ein oberes Ende (2044) des Ventilkörpers (204) an der ringförmigen Stufe (2036) anliegt und zwischen der ringförmigen Stufe (2036) und dem Sprühloch (2037) ein Spalt ausgebildet ist, und ein unteres Ende des Ventil körpers (204) an der Feder (204) anliegt.

6. Zerstäubungsvorrichtung nach Anspruch 4, wobei der Ventilkörper (204) einen oberen Ventilkörper (2041), einen unteren Ventilkörper (2042) und einen Ventilwulst (2043) aufweist, wobei ein Teil des unteren Ventils (2042) in dem oberen Ventilkörper (2041) untergebracht ist, wobei der Ventilwulst (2043) zwischen dem oberen Ventil (2041) und unterer Ventilkörper (2042) angeordnet ist, der untere Ventilkörper (2042) hohl ist und in Verbindung mit dem Saugrohr (205) steht, wobei der obere Ventilkörper (2041) und der untere Ventilkörper (2042) gemeinsam einen Spalt definierten.

7. Zerstäubungsvorrichtung nach Anspruch 1, wobei das Heizelement (104) aus poröser Keramik oder porösem Metall hergestellt ist.

8. Zerstäubungsvorrichtung nach Anspruch 1, ferner umfassend ein Mundstück (107), wobei das Mundstück (107) und die Sprühvorrichtung (20) an zwei gegenüberliegenden Seiten des Heizelements (104) angeordnet sind.

9. Zerstäubungsvorrichtung nach Anspruch 1, ferner umfassend eine Taste (110; 111), die zum Einstellen einer Ausgangsleistung der Stromversorgung (105) konfiguriert ist.

## Revendications

1. Dispositif d'atomisation par pulvérisation (10), comprenant :
un boîtier (101), le boîtier (101) définissant une chambre d'atomisation (102) ;
un réservoir de liquide (103) conçu pour le stockage du liquide de tabac ;
un dispositif de pulvérisation (20) conçu pour pulvériser le liquide de tabac dans la chambre d'atomisation (102) sous une forme de particules liquides ;
un élément chauffant (104) agencé dans la chambre d'atomisation (102), l'élément chauffant (104) présentant une surface de chauffage (1041), la surface de chauffage (1041) étant conçue pour soutenir et chauffer les particules liquides pour former un aérosol ; et
un bloc d'alimentation (105) agencé dans le boîtier (101), le bloc d'alimentation (105) étant conçu pour fournir l'alimentation à l'élément chauffant ;
**caractérisé en ce que** le dispositif d'atomisation (10) comprend en outre une chambre de réception (106) dans le boîtier (101) en dessous de l'élément chauffant (104), dans lequel la chambre de réception (106) est en communication avec le réservoir de liquide (103), de manière à ce que les particules liquides, qui ne sont pas atomisées, puissent s'écouler dans la chambre de réception (106), puis dans le réservoir de liquide (103).

2. Dispositif d'atomisation selon la revendication 1, dans lequel la surface de chauffage (1041) se trouve en regard du dispositif de pulvérisation (20) et est inclinée par rapport à la direction horizontale.

3. Dispositif d'atomisation selon la revendication 1, dans lequel le dispositif de pulvérisation (20) comprend une buse (201), une tête de pression (202), un corps de pompe (203) et un tube d'aspiration (205) raccordé à une extrémité du corps de pompe (203), le corps de pompe (203) est en communication avec le liquide de tabac dans le réservoir de liquide (103) par l'intermédiaire du tube d'aspiration (205), une pression d'air dans le corps de pompe (203) peut être modifiée en appuyant sur la tête de pression (202), de sorte que du liquide de tabac peut être pulvérisé et atomisé par l'intermédiaire de la buse (201).

4. Dispositif d'atomisation selon la revendication 3, dans lequel le corps de pompe (203) comprend un corps principal (2032), un piston (2031) dans le corps (2032) un corps de vanne (204), le piston (2031) est creux et en communication avec la buse (201), le piston (2031) et le corps principal (2032) définissent conjointement une chambre de compression (2033), le corps de vanne (204) est agencé dans la chambre de compression (2033) et conçu pour commander l'entrée de liquide de tabac dans la chambre de compression (2033).

5. Dispositif d'atomisation selon la revendication 4, dans lequel le corps de pompe (203) comprend en outre un étage annulaire (2036) et un ressort (2034), l'étage annulaire (2036) forme un trou de pulvérisation (2037), une extrémité supérieure (2044) du corps de vanne (204) vient en butée contre l'étage annulaire (2036) et un espace est formé entre l'étage annulaire (2036) et l'orifice de pulvérisation (2037) et une partie d'extrémité inférieure du corps de vanne (204) vient en butée contre le ressort (2034).

6. Dispositif d'atomisation selon la revendication 4, dans lequel le corps de vanne (204) comprend un corps de vanne supérieur (2041), un corps de vanne inférieur (2042) et une bille de vanne (2043), la partie du corps de vanne inférieur (2042) étant nichée dans le corps de vanne supérieur (2041), la bille de vanne (2043) est intercalée entre le corps de vanne supérieur (2041) et le corps de vanne inférieur (2042), le corps de vanne inférieur (2042) est creux et en communication avec le tube d'aspiration (205), le corps de vanne supérieur (2041) et le corps de vanne inférieur (2042) définissent conjointement un espace.

7. Dispositif d'atomisation selon la revendication 1, dans lequel l'élément chauffant (104) est fabriqué en céramique poreuse ou en métal poreux.

8. Dispositif d'atomisation selon la revendication 1, comprenant en outre une embouchure (107), dans lequel l'embouchure (107) et le dispositif de pulvérisation (20) sont agencés à deux côtés opposés de l'élément chauffant (104).

9. Dispositif d'atomisation selon la revendication 1, comprenant en outre un bouton (110, 111) conçu pour régler une puissance de sortie du bloc d'alimentation (105).
